# EUROPEAN PATENT SPECIFICATION

(11) **EP 0 352 856 B1**
(45) Date of publication and mention of the grant of the patent: **13.01.1993**
(21) Application number: 89201925.8
(22) Date of filing: 21.07.1989
(51) Int. Cl.: C07C 2/26, B01J 31/02

(54) **Process of selective oligomerization of olefins and new catalyst for such a process**
Verfahren zur selektiven Oligomerisierung von Olefinen und Katalysator für ein solches Verfahren
Procédé pour l'oligomérisation sélective d'oléfines et catalyseur pour un tel procédé

(30) Priority: 29.07.1988 IT 2155188
(43) Date of publication of application: 31.01.1990
(73) Proprietor: Enichem Anic S.r.l., 90139 Palermo (IT)
(72) Inventor: Messina, Giuseppe, I-07041 Alhero Sassari (IT); Lorenzoni, Loreno, I-07046 Porto Torres Sassari (IT); Virdis, Angelo, I-07049 Usini Sassari (IT); Orlandoni, Anna Maria, I-07041 Alghero Sassari (IT)
(74) Representative: Roggero, Sergio

(56) References cited:
- EP-A- 0 125 769
- US-A- 3 842 019
- CHEMICAL ABSTRACTS, vol. 95, no. 21, May 23, 1983, Columbus, Ohio, USA HIGASHIMURA, TOSHINOBU et al.:"Selective synthesis of iso- butene trimers and hexamers with oxo acid catalysts." page 596, abstract no. 178 673h
- CHEMICAL ABSTRACTS, vol. 101, no. 1, July 2, 1984, Columbus, Ohio, USA HIGASHIMURA, TOSHINOBU: "C4-Olefin trimers." page 562, abstract no. 6 597a
- EUROPEAN POLYMER JOURNAL, vol. 16, 1980, Oxford J. COLLOMB et al. "Cationic polymerization induced by metal salts-I" pages 1135-1144

## Description

The present invention relates to a process for the selective oligomerization of olefins containing from 3 to 6 carbon atoms, and to the heterogeneous-phase, acidic catalyst used in said process.

In particular, the present invention relates to a process of oligomerization of C₃-C₆ olefins under conditions of heterogeneous-phase acidic catalysis, wherein as the catalyst a salt of trifluoromethanesulfonic acid (triflic acid) with particular metals, or a mixture of such salts, dispersed on a chemically and thermally stable support having a large surface area, is used.

The oligomers of C₃-C₆ olefins are extremely interesting compounds from an industrial point of view. In particular, e.g., an olefinic oligomer containing 8 carbon atoms, either straight or with a branching constituted by a methyl group, is used in the field of plasticizers (nonyl phthalate), and of the fuels for diesel engines and reaction engines, while the oligomer containing the same number of carbon atoms (8), but with a more branched chain, is used in high antiknock-power gasolines. Oligomers containing twelve carbon atoms are useful intermediates in the field of detergents, of lubricants, of perfumes, of dyes, of resins, and so forth.

Therefore, several specific catalysts for the oligomerization of olefins under conditions of heterogenous catalysis were used. Among these, those catalysts can be cited, which are obtained by impregnating a metal oxide containing hydroxy groups, pre-calcined at 300-500°C, with titanium tetrafluoride or titanium tetrachloride (U.S. patents Nos. 4,108,920 and 4,110,410) or intercalating antimony fluoride into graphite (U.S. patent No. 4,463,212), or, also, by supporting phosphorus fluoride on gamma-alumina (U.S. patent No. 4,465,885), and fluorinated aluminum phosphate (U.S. patent No. 4,476,342).

Unfortunately, no one of these catalysts supplies a totally satisfactory performance, in that all of them operate either with poor conversions of the olefins used as the starting products, or with poor selectivity, yielding mixtures of products with a too wide range of distribution of the constituting isomers, or, moreover, they are active at too high temperatures with low specificity towards the desired products, or, finally, they require extremely long stay times, with consequent low productivity values.

The process according to the present invention, which uses one or more salts of triflic acid dispersed on a large surface-area support as the heterogenous-phase acidic catalyst, makes it possible, on the contrary, for oligomers (dimers, trimers or tetramers) of C₃-C₆ olefins to be obtained by operating under mild temperature and pressure conditions, and with a high productivity.

Such a result is still more surprising, if one considers that the study recently performed by J. Collomb, B. Morin, A. Gandini and H. Cheradame [European Polymer Journal, Volume 16, 1135-44 (1980)], showed that the use of salts of triflic acid in the cationic polymerization of olefins, and, mainly, of isobutene, in a vehicle in which such salts are not soluble (and therefore under heterogeneous catalysis conditions), leads to polymers, the molecular weight of which depends on the reaction conditions, but which are always constituted by more than 15 monomeric units.

The C₃-C₆ olefins which can be used as the starting material in the process according to the present invention preferably are straight olefins belonging to the group of the alpha-olefins and of the straight olefins with internal double bonds.

Linear olefins which are used as the starting material are propylene, butene-1, butene-2, pentene-1, pentene-2, hexene-1, hexene-2 and hexene-3.

However, to the process of oligomerization according to the present invention also the branched isomers of such olefins can be submitted; these will anyway originate more branched oligomers, with lower productivity values.

According to a particularly preferred form of practical embodiment of the present invention, the reaction of oligomerization is carried out by starting from butene-2 or from mixtures of butene-1 and butene-2. As already said, in fact, the oligomers of 8 carbon atoms and of 12 carbon atoms are those which deserve most interest at an industrial level.

Furthermore, the development of large-scale production of methyl-tert.-butyl-ether (MTBE), which is manufactured at present by reacting isobutene contained in C₄ cut (a blend of C₄ olefins and paraffins) with methanol, caused large amounts of straight butenes to become available, which at present are not used for any major applications.

The oligomers obtained by means of the process according to the present invention are dimers, trimers and tetramers of the starting olefin, containing from 6 to 24 carbon atoms.

The term "oligomerization" defines therefore the reaction of an olefinic monomer with itself, with a dimer thereof, or with a trimer thereof, or, in case a plurality of olefins are used as the starting product, with a different olefinic monomer, or with a dimer or a trimer of this latter.

The catalytic system according to the present invention used for the oligomerization of such olefins comprises one or more salt(s) of trifluoromethanesulfonic acid with metals of the IInd, IIIrd, IVth and Vth Group, or with transition metals, dispersed on a chemically and thermally stable support endowed with a large surface area.

Among the salts of trifluoromethane-sulfonic acid (in a shorter form: "triflic acid"), useable in the process according to the present invention, magnesium triflate, barium triflate, aluminum triflate, gallium triflate, germanium triflate, tin triflate, lead triflate, antimony triflate, bismuth triflate, scandium triflate, chrome triflate, iron triflate, cobalt triflate, nickel triflate, copper triflate, zinc triflate, yttrium triflate, zirconium triflate, molibdenum triflate, cadmium triflate, cerium triflate, and so forth can be mentioned.

For the purposes of the present invention, within the term of "salts of triflic acid with a metal of the IInd, IIIrd, IVth and Vth Group, or with a transition metal", also those mixed salts are included, which contain such a metal, triflate ion and a further anion, such as, e.g., a halide. From the group of these mixed salts, the triflate-chlorides (-bromides and -iodides) of aluminum and gallium can be mentioned for exemplifying purposes.

The salts of triflic acid which can be used in the process according to the present invention can be prepared by means of conventional methods easily found by those skilled in the art.

Some of these salts are products available from the market. Anyway, a general synthesis route provides for the carbonates or the hydroxides of the corresponding metals to be treated with aqueous triflic acid.

In some cases, for example for the preparation of aluminum triflate or of zirconium triflate, the metal powder or metal chips can be directly attacked with an aqueous solution of triflic acid.

According to other methods of preparation of these salts, a halide of the metal can be treated with triflic acid in an organic, polar, aprotic solvent, such as, e.g., a cyclic ether, for instance, dioxane, tetrahydrofuran, a halogenated aliphatic hydrocarbon, for instance methylene chloride, dichloroethane, or other similar solvents, or an alkyl-metal to be treated with triflic acid, also in an organic vehicle.

The salt of triflic acid which is obtained in that way is then dispersed on a chemically and thermally stable support having a large surface area.

Suitable supports are all those supports, whether porous or non-porous, which are endowed with a large surface area, and with a considerably high thermal and chemical stability.

In particular, examples of supports which can be well used in the process according to the present invention are vegetable charcoal, and, in particular, activated charcoal (a microcrystalline, non-graphitic form of charcoal, so treated as to develop an inner porosity), graphite, and the metal oxides, such as gamma-alumina, titanium dioxide (anatase and rutile), silica, and the either natural or synthetic silica-alumina blends having a large surface area.

By means of the term "support having a large surface area", those supports are meant, which have a surface area comprised within the range of from 5 to 2000 m²/g and, preferably, comprised within the range of from 50 to 1200 m²/g.

The supports used can be in the form of a powder, of granules, of microspheres, of pellets, of extruates, and so forth.

Such supports can be, and preferably are, dehydrated by calcination at temperatures comprised within the range of from about 200 to about 600°C. The salts of triflic acid are then dispersed on the support according to known methods.

A general method consists in impregnating the support with the selected salt(s), by pouring a solution of these latter on said support up to "incipient wetting" of this latter, and then evaporating off the solvent, with the consequent immobilization of the triflate on the support, or by dipping the support into the solution of the triflate, and then evaporating the solvent. The evaporation step is usually carried out by heating, possibly under vacuum. If desired, the support wetting/solvent evaporation cycle can be repeated a plurality of times.

The impregnation and the evaporation of the solvent can be carried out also simultaneously, by dropwise adding a triflate solution to the support, with this latter being suitably heated and kept stirred inside a suitable vessel. When the salt(s) of triflic acid is(are) directly obtained in solution (for instance, when an aqueous solution of triflic acid is treated with the metal powder), such solutions can be directly used for impregnating the support. When the preparation of the salt(s) is carried out, on the contrary, in one or more organic solvent(s), the jelly-like suspension which is obtained can be dispersed on the support by coating or soaking.

According to an alternative route, the desired salt of triflic acid can be directly formed on the support.

In particular, in such case, once that the selected support is impregnated with triflic acid (which impregnation is obtained by directly wetting the support with triflic acid or with a solution of this latter in a suitable solvent and then evaporating off this latter), metal in the form of an alkyl-metal is then made to flow above it. If such an alkyl-metal is volatile enough, it will be made to flow over the support -- impregnated with triflic acid -- in the gas phase, otherways as a solution in a suitable inert organic solvent.

An alternative route for preparing the heterogeneous-phase catalyst according to the present invention consists in intimately mixing the preselected salt(s) of triflic acid, as a fine powder, with the support, also as a fine powder, with said blend being then tabletted or pelletized.

The total amount of salts of triflic acid dispersed on the support is generally comprised within the range of from 1 to 50% by weight, as referred to the total resulting weight. However, such an amount will be preferably comprised within the range of from 5 to 40% by weight, relatively to the total resulting weight, and still more preferably, it will be comprised within the range of from 10 to 30%.

Once that the dispersion of the salt(s) of triflic acid on the support is obtained, the so prepared catalyst is dried and calcined at temperatures typically comprised within the range of from 50 to 350°C.

The so-obtained new catalysts, besides having shown to be very good catalysts of oligomerization at relatively low temperatures, are endowed -- as compared to other catalysts known in the field from the prior art, such as, e.g., supported AlCl₃, BF₃, TiCl₄ -- with the advantageous characteristic that they have a higher stability to temperature (in that they generally are higher-boiling than chlorine and fluorine salts) and to hydrolysis.

The novel catalysts have shown to be particularly useful in the selective oligomerization of C₃-C₆ olefins.

The invention, therefore, provides a process for oligomerizing C₃ to C₆ olefins, which comprises oligomerizing the olefin concerned in the presence of a heterogeneous-phase acidic catalyst, at a temperature of from 50°C to 200°C and under a pressure of from 0,4 MPa (4 atm) to 10 MPa (100 atm), which is characterized in that said catalyst is a system comprising a trifluoromethanesulphonic acid salt, or a trifluoromethanesulphonic acid halide salt, of a metal selected from the metals of the II-Group, the III-Group, the IV-Group and the Vth-Group and the transition metal Group of the Periodic Table, or any admixture thereof, and is supported, in dispersed form, throughout the mass of a chemically and thermally stable solid support having a surface area of from 5 m²/g to 2000m²/g. It is preferred that the oligomerization reaction temperature is from 100°C to 170°C, according to which olefin is used as the feedstock, and that oligomerization reaction is carried out under a pressure of from 0,5 MPa to 6 MPa (5 atm-60 atm).

When the reaction of oligomerization is carried out under these preferred conditions, by starting from a C₄ olefin, oligomers containing 8 carbon atoms (mainly methyl-heptene and dimethyl-hexene, from butene-1 and/or butene-2) can be selectively obtained with high conversions and productivities.

The reaction of oligomerization according to the present invention can be carried out as a continuous reaction, or also batchwise.

When the reaction, according to a preferred form of practical embodiment of the invention, is carried out as a continuous process, both the technique of the stationary bed, and the technique of the moving bed can be used.

In the first case, a determined amount of catalyst is charged to a reactor suitabte for operating under preselected temperature and pressure conditions, with the catalyst being preferably kept under an inert blanketing atmosphere, e.g., under nitrogen or argon, and an amount of olefinic hydrocarbon generally comprised within the range of from 0.5 to 10 kg of olefin/kg of catalyst per hour being continuously flown over the catalyst bed.

After a predetermined time of contact with the catalytic bed, which may be comprised within the range of from 0.1 up to about 10 hours, the effluent is continuously discharged and is submitted to the conventional separation methods (e.g., by fractional distillation) in order to recover the formed oligomers, and recycle any unreacted olefins to the reactor feed.

If so desired, the olefin can be fed as a mixture with a paraffinic hydrocarbon acting as a diluent. In case, for example, the stream is used, which derives from the C₄ cut from which isobutene is removed, and which contains, besides the mixture of straight butenes, also such paraffins as butane (and/or isobutane), such a stream can be advantageously fed in its pristine state, without requiring a preliminary separation step.

In case the moving-bed technique is used, the catalyst and the feed stream are made flow, in co-current flow or counter-current flow mode, through the reactor. As regards the contact times, the ratio between the olefin and the catalyst, and the feed type, the same considerations as made above are valid.

A third method by means of which the oligomerization can be carried out as a continuous process using the catalyst according to the present invention consists of charging the catalyst to the reactor as a suspension in the feed liquid.

The following examples are supplied in order to better illustrate the present invention as practically embodied according to some representative forms thereof, and should not be regarded as a limitation for the purposes of the same invention.

### Example 1

A catalyst for the process according to the present invention is prepared by charging AlCl₃ (6.867 g, 0.0515 mol) and Freon^{(R)} 113 (100 ml) to a flask of 250 ml, under a nitrogen stream. To this solution, kept with stirring and cooled at about 0°C, trifluoromethanesulfonic acid is added (23.18 g, 0.1545 mol). The development of HCl is observed. The reaction mixture is kept with stirring at room temperature for about 12 hours, and the jelly-like suspension which is obtained is then washed with Freon^{(R)} 113 (3 times, with 50 ml each time). The obtained product is gradually heated, under a nitrogen stream, up to 110°C, and is maintained at this temperature 2 hours long. The so obtained aluminum triflate (24.41 g) is dissolved, with stirring, in a mixture of methylene chloride (900 ml) and tetrahydrofuran (14.83 g). To the solution, artivated charcoal is added (97.64 g), and the solution is maintained at 40°C, with stirring, for 3 hours. The solvent is then evaporated off by slow, gradual heating, under a nitrogen atmosphere, up to 80°C. The so obtained product, still under nitrogen, is heated up to 150°C, and is maintained at this temperature for about 2 hours. A portion (30 ml, 7.5 g) of the so obtained catalyst is charged to a fixed-bed reactor under a nitrogen stream. By operating at the temperature of 120°C, and under the pressure of 5 MPa (50 atm), over the catalyst a mixture of butene-2 (60%) and butane (40%) is flown, with a contact time (expressed as litres of catalyst per each liter of fed mixture per hour) of 1.

The reaction product is then collected and examined. A conversion per butene-2 passage of 61.3%, and a selectivity to octenes of 82% are obtained, with the following internal distribution of octene isomers :
- 12.5% of 2-methyl-heptene,
- 86.2% of dimethyl-hexenes, and
- 1.3% of trimethyl-pentenes.

### Example 2

Aluminum powder (2.8 g) is slowly added (2 hours) to a solution of trifluoromethane-sulfonic acid (15 g, 0.1 mol) in water (100 ml), charged under a nitrogen stream to a flask of 250 ml of capacity, and kept at 21°C with vigorous stirring.

The reaction mixture is then gradually heated, still under nitrogen, up to the reflux temperature, and is maintained under a slow reflux for 1 hour until the metal is completely dissolved. A portion (65 ml) of the obtained jelly-like product, completely soluble at 80°C, is poured at this temperature on gamma-alumina (163 g), pre-treated at 400°C for 2 hours. So treated gamma-alumina is slowly dried under a nitrogen atmosphere by heating up to 110°C, then the remainder portion (35 ml) of aluminum triflate containing solution is poured at 80°C on the alumina. The drying step is repeated by means of a further 2 hours of heating at 110°C under a nitrogen stream, then the alumina is calcined, still under a nitrogen stream, for 1 hours at 200°C.

The so obtained catalyst is charged to a fixed-bed reactor under an inert atmosphere consisting of dry nitrogen. The reactor temperature is subsequently heated up to 150°C. The pressure inside the reactor is then increased up to 5 MPa (50 atm), and a mixture composed by 60% of butene-2 and 40% of butane is made flow through it, with a stay time (expressed as litres of catalyst/litre of fed mixture per hour) of 1.

The reaction product is collected and analysed, and the following results are obtained:
- conversion per butene-2 passage = 53.4%;
- selectivity to octenes = 91,3%;
   with the following internal distribution of octene isomers:
- 8.7% of 2-methyl-heptene,
- 85.8% of dimethyl-hexenes, and
- 1.8% of trimethyl-pentenes.

## Claims (Claims for the following Contracting State(s): AT, BE, CH, DE, FR, GB, LI, LU, NL, SE)

1. A process for oligomerizing C₃ to C₆ olefins, which comprises oligomerizing the olefin concerned in the presence of a heterogeneous-phase acidic catalyst, at a temperature of from 50°C to 200°C and under a pressure of from 0,4 MPa (4 atm) to 10 MPa (100 atm), characterized in that said catalyst is a system comprising a trifluoromethanesulphonic acid salt, or a trifluoromethanesulphonic acid halide salt, of a metal selected from the metals of the II-Group, the III-Group, the IV-Group and the Vth-Group and the transition metal Group of the Periodic Table, or any admixture thereof, and is supported, in dispersed form, throughout the mass of a chemically and thermally stable solid support having a surface area of from 5 m²/g to 2000m²/g.

2. Process according to Claim 1, wherein the oligomerization reaction temperature is from 100°C to 170°C.

3. Process according to Claim 1, wherein the oligomerization reaction is carried out under a pressure of from 0,5 MPa to 6 MPa (5 atm-60 atm).

4. Process according to Claim 1, wherein the olefin concerned is a straight-chain olefin.

5. Process according to Claim 4, wherein the olefin concerned is a C₄-straight-chain olefin.

6. Process according to Claim 1, wherein the surface area of the support is from 50 m²/g to 1200 m²/g.

7. Process according to Claim 1, wherein the support is selected from vegetable charcoal, graphite, and metal oxides.

8. Process according to Claim 7, wherein the metal oxide is selected from gamma-alumina; titanium dioxide in anatase or rutile form, silica, and naturally occurring or synthesized silica-alumina mixtures.

9. Process according to Claim 1, wherein the trifluoromethanesulphonic acid salt is present in the supported catalyst in an amount of from 1% to 50% by weight.

10. Process according to Claim 9, wherein the trifluoromethanesulphonic acid salt is present in the supported catalyst in an amount of from 5% to 40% by weight.

11. Process according to Claim 10, wherein the trifluoromethanesulphonic acid salt is present in the supported catalyst in an amount of from 10% to 30% by weight.

12. Process according to Claim 1, wherein the metal in the trifluoromethanesulphonic acid salt is selected from: magnesium, barium, aluminium, gallium, tin, lead, antimony, bismuth, scandium, chromium, iron, cobalt, nickel, copper, zinc, yttrium, zirconium, molybdenum, cadmium and cerium.

13. Process according to Claim 1, wherein the trifluoromethanesulphonic acid salt is aluminium trifluoromethanesulphonate.

14. Process according to Claim 1, wherein the trifluoromethanesulphonic acid halide salt is an aluminium salt of a trifluoromethanesulphonic acid halide.

15. Process according to Claim 1, wherein the olefin concerned is fed to the oligomerization reaction in admixture with a paraffinic hydrocarbon as the diluent.

16. A heterogeneous-phase catalyst system for oligomerizing olefins, characterized in that it comprises a trifluoromethanesulphonic acid salt, or a trifluoromethanesulphonic acid halide salt, of a metal selected from the metals of the II-Group, the III-Group, the IV-Group and the Vth-Group and the transition metal Group of the Periodic Table, or any admixture thereof, and is supported in dispersed form throughout the mass of a chemically and thermally stable solid support having a surface area of from 5 m²/g to 2000 m²/g.

17. Catalyst system according to Claim 16, wherein the metal oxide is selected from gamma-alumina, titanium dioxide in anatase or rutile form, silica, and naturally occurring or synthesized silica-alumina mixtures.

18. Catalyst system according to Claim 16, wherein the metal is aluminium.

## Claims (Claims for the following Contracting State(s): ES)

1. A process for oligomerizing C₃ to C₆ olefins, which comprises oligomerizing the olefin concerned in the presence of a heterogeneous-phase acidic catalyst, at a temperature of from 50°C to 200°C and under a pressure of from 0,4 MPa (4 atm) to 10 MPa (100 atm), characterized in that said catalyst is a system comprising a trifluoromethanesulpbonic acid salt, or a trifluoromethanesulphonic acid halide salt, of a metal selected from the metals of the II-Group, the III-Group, the IV-Group and the Vth-Group and the transition metal Group of the Periodic Table, or any admixture thereof, and is supported, in dispersed form, throughout the mass of a chemically and thermally stable solid support having a surface area of from 5 m²/g to 2000 m²/g.

2. Process according to Claim 1, wherein the oligomerization reaction temperature is from 100°C to 170°C.

3. Process according to Claim 1, wherein the oligomerization reaction is carried out under a pressure of from 0,5 MPa to 6 MPa (5 atm-60 atm).

4. Process according to Claim 1, wherein the olefin concerned is a straight-chain olefin.

5. Process according to Claim 4, wherein the olefin concerned is a C₄-straight-chain olefin.

6. Process according to Claim 1, wherein the surface area of the support is from 50 m²/g to 1200 m²/g.

7. Process according to Claim 1, wherein the support is selected from vegetable charcoal, graphite, and metal oxides.

8. Process according to Claim 7, wherein the metal oxide is selected from gamma-alumina, titanium dioxide in anatase or rutile form, silica, and naturally occurring or synthesized silica-alumina mixtures.

9. Process according to Claim 1, wherein the trifluoromethanesulphonic acid salt is present in the supported catalyst in an amount of from 1% to 50% by weight.

10. Process according to Claim 9, wherein the trifluoromethanesulphonic acid salt is present in the supported catalyst in an amount of from 5% to 40% by weight.

11. Process according to Claim 10, wherein the trifluoromethanesulphonic acid salt is present in the supported catalyst in an amount of from 10% to 30% by weight.

12. Process according to Claim 1, wherein the metal in the trifluoromethanesulphonic acid salt is selected from: magnesium, barium, aluminium, gallium, tin, lead, antimony, bismuth, scandium, chromium, iron, cobalt, nickel, copper, zinc, yttrium, zirconium, molybdenum, cadmium and cerium.

13. Process according to Claim 1, wherein the trifluoromethanesulpbonic acid salt is aluminium trifluoromethanesulphonate.

14. Process according to Claim 1, wherein the trifluoromethanesulphonic acid halide salt is an aluminium salt of a trifluoromethanesulphonic acid halide.

15. Process according to Claim 1, wherein the olefin concerned is fed to the oligomerization reaction in admixture with a paraffinic hydrocarbon as the diluent.

## Patentansprüche (Patentansprüche für folgende(n) Vertragsstaat(en): AT, BE, CH, DE, FR, GB, GR, LI, LU, NL, SE)

1. Verfahren zum Oligomerisieren von C₃- bis C₆-Olefinen, welches ein Oligomerisieren des betreffenden Olefins in Anwesenheit eines heterogenphasigen sauren Katalysators bei einer Temperatur von 50 bis 200°C und unter einem Druck von 0,4 MPa (4 Atmosphären) bis 10 MPa (100 Atmosphären) umfaßt, dadurch gekennzeichnet, daß der Katalysator ein System ist, das ein Trifluormethansulfonsäuresalz oder ein Trifluormethasulfonsäurehalogenidsalz eines unter den Metallen der Gruppe II, Gruppe III, Gruppe IV und Gruppe V und der Übergangsmetallgruppe des Periodensystems der Elemente ausgewählten Metalles oder ein Gemisch hievon umfaßt und in dispergierter Form durch die Masse eines chemisch und thermisch stabilen festen Trägers mit einer Oberfläche von 5 m²/g bis 2.000 m²/g gestützt ist.

2. Verfahren nach Anspruch 1, worin die Oligomerisationsreaktionstemperatur von 100°C bis 170°C beträgt.

3. Verfahren nach Anspruch 1, worin die Oligomerisationsreaktion unter einem Druck von 0.5 MPa bis 6 MPa (5 Atmosphären bis 60 Atmosphären) ausgeführt wird.

4. Verfahren nach Anspruch 1, worin das betreffende Olefin ein geradkettiges Olefin ist.

5. Verfahren nach Anspruch 4, worin das betreffende Olefin ein C₄-geradkettiges Olefin ist.

6. Verfahren nach Anspruch 1, worin die Oberfläche des Trägers 50 m²/g bis 1.200 m²/g beträgt.

7. Verfahren nach Anspruch 1, worin der Träger unter Pflanzenkohle, Graphit und Metalloxiden ausgewählt ist.

8. Verfahren nach Anspruch 7, worin das Metalloxid unter gamma-Aluminiumoxid, Titandioxid in Anatas- oder Rutilform, Siliziumdioxid und natürlich vorkommenden oder synthetisierten Siliziumdioxid-Aluminiumoxid-Gemischen ausgewählt ist.

9. Verfahren nach Anspruch 1, worin das Trifluormethansulfonsäuresalz im Trägerkatalysator in einer Menge von 1 bis 50 Gew.-% vorliegt.

10. Verfahren nach Anspruch 9, worin das Trifluormethansulfonsäuresalz im Trägerkatalysator in einer Menge von 5 bis 40 Gew.-% vorliegt.

11. Verfahren nach Anspruch 10, worin das Trifluormethansulfonsäuresalz im Trägerkatalysator in einer Menge von 10 bis 30 Gew.-% vorliegt.

12. Verfahren nach Anspruch 1, worin das Metall im Trifluormethansulfonsäuresalz unter Magnesium, Barium, Aluminium, Gallium, Zinn, Blei, Antimon, Bismut, Scandium, Chrom, Eisen, Kobalt, Nickel, Kupfer, Zink, Yttrium, Zirkonium, Molybdän, Cadmium und Cer ausgewählt ist.

13. Verfahren nach Anspruch 1, worin das Trifluormethansulfonsäuresalz Aluminiumtrifluormethansulfonat ist.

14. Verfahren nach Anspruch 1, worin das Trifluormethansulfonsäurehalogenidsalz ein Aluminimsalz eines Trifluormethansulfonsäurehalogenids ist.

15. Verfahren nach Anspruch 1, worin das betreffende Olefin im Gemisch mit eimem Paraffinkohlenwasserstoff als Verdünnungsmittel der Oligomerisationsreaktion zugeführt wird.

16. Heterogenphasiges Katalysatorsystem zum Oligomerisieren von Olefinen, dadurch gekennzeichnet, daß es ein Trifluormethansulfonsäuresalz oder ein Trifluormethansulfonsäurehalogneidsalz eines unter den Metallen der Gruppe II, Gruppe III, Gruppe IV, Gruppe V und der Übergangsmetallgruppe des Periodensystems der Elemente ausgewählten Metalles oder ein Gemisch hievon umfaßt und in dispergierter Form durch die Masse eines chemisch und thermisch stabilen festen Trägers mit einer Oberfläche von 5 m²/g bis 2.000 m²/g verteilt ist.

17. Katalysatorsystem nach Anspruch 16, worin das Metalloxid unter gamma-Aluminiumoxid, Titandioxid in Anatas- oder Rutilform, Siliziumdioxid und natürlich vorkommenden oder synthetisierten Siliziumdioxid-Aluminiumoxid-Gemischen ausgewählt ist.

18. Katalysatorsystem nach Anspruch 16, worin das Metall Aluminium ist.

## Patentansprüche (Patentansprüche für folgende(n) Vertragsstaat(en): ES)

1. Verfahren zum Oligomerisieren von C₃- bis C₆-Olefinen, welches ein Oligomerisieren des betreffenden Olefins in Anwesenheit eines heterogenphasigen sauren Katalysators bei einer Temperatur von 50 bis 200°C und unter einem Druck von 0,4 MPa (4 Atmosphären) bis 10 MPa (100 Atmosphären) umfaßt, dadurch gekennzeichnet, daß der Katalysator ein System ist, das ein Trifluormethansulfonsäuresalz oder ein Trifluormethansulfonsäurehalogenidsalz eines unter den Metallen der Gruppe II, Gruppe III, Gruppe IV und Gruppe V und der Übergangsmetallgruppe des Periodensystems der Elemente ausgewählten Metalles oder ein Gemisch hievon umfaßt und in dispergierter Form durch die Masse eines chemisch und thermisch stabilen festen Trägers mit einer Oberfläche von 5 m²/g bis 2.000 m²/ gestützt ist.

2. Verfahren nach Anspruch 1, worin die Oligomerisationsreaktionstemperatur von 100°C bis 170°C beträgt.

3. Verfahren nach Anspruch 1, worin die Oligomerisationsreaktion unter einem Druck von 0.5 MPa bis 6 MPa (5 Atmosphären bis 60 Atmosphären) ausgeführt wird.

4. Verfahren nach Anspruch 1, worin das betreffende Olefin ein geradkettiges Olefin ist.

5. Verfahren nach Anspruch 4, worin das betreffende Olefin ein C₄-geradkettiges Olefin ist.

6. Verfahren nach Anspruch 1, worin die Oberfläche des Trägers 50 m²/g bis 1.200 m²/g beträgt.

7. Verfahren nach Anspruch 1, worin der Träger unter Pflanzenkohle, Graphit und Metalloxiden ausgewählt ist.

8. Verfahren nach Anspruch 7, worin das Metalloxid unter gamma-Aluminiumoxid, Titandioxid in Anatas- oder Rutilform, Siliziumdioxid und natürlich vorkommenden oder synthetisierten Siliziumdioxid-Aluminiumoxid-Gemischen ausgewählt ist.

9. Verfahren nach Anspruch 1, worin das Trifluormethansulfonsäuresalz im Trägerkatalysator in einer Menge von 1 bis 50 Gew.-% vorliegt.

10. Verfahren nach Anspruch 9, worin das Trifluormethansulfonsäuresalz im Trägerkatalysator in einer Menge von 5 bis 40 Gew.-% vorliegt.

11. Verfahren nach Anspruch 10, worin das Trifluormethansulfonsäuresalz im Trägerkatalysator in einer Menge von 10 bis 30 Gew.-% vorliegt.

12. Verfahren nach Anspruch 1, worin das Metall im Trifluormethansulfonsäuresalz unter Magnesium, Barium, Aluminium, Gallium, Zinn, Blei, Antimon, Bismut, Scandium, Chrom, Eisen, Kobalt, Nickel, Kupfer, Zink, Yttrium, Zirkonium, Molybdän, Cadmiun und Cer ausgewählt ist.

13. Verfahren nach Anspruch 1, worin das Trifluormethansulfonsäuresalz Aluminiumtrifluormethansulfonat ist.

14. Verfahren nach Anspruch 1, worin das Trifluormethansulfonsäurehalogenidsalz ein Aluminimsalz eines Trifluormethansulfonsäurehalogenids ist.

15. Verfahren nach Anspruch 1, worin das betreffende Olefin im Gemisch mit einem Paraffinkohlenwasserstoff als Verdünnungsmittel des Oligomerisationsreaktion zugeführt wird.

## Revendications (Revendications pour l'(les) Etat(s) contractant(s) suivant(s): AT, BE, CH, DE, FR, GB, GR, LI, LU, NL, SE)

1. Procédé d'oligomérisation d'oléfines en C₃ à C₆, comprenant l'oligomérisation de l'oléfine concernée en présence d'un catalyseur acide, en phase hétérogène, à une température de 50°C à 200°C et sous une pression de 0,4 MPa (4 atm) à 10 MPa (100 atm), **caractérisé** en ce que ledit catalyseur est un système comprenant un trifluorométhanesulfonate ou un halogénure-trifluorométhanesulfonate d'un métal choisi parmi les métaux du groupe II, du groupe III, du groupe IV, du groupe V et du groupe des métaux de transition du Tableau Périodique, ou un mélange quelconque de ces composés, et est fixé, sous forme dispersée, dans toute la masse d'un support solide, chimiquement et thermiquement stable, ayant une surface spécifique de 5 m² /g à 2 000 m² /g.

2. Procédé selon la revendication 1, dans lequel la température de la réaction d'oligomérisation est de 100°C à 170°C.

3. Procédé selon la revendication 1, dans lequel on effectue la réaction d'oligomérisation sous une pression de 0,5 MPa à 6 MPa (5 atm à 60 atm).

4. Procédé selon la revendication 1, dans lequel l'oléfine concernée est une oléfine à channe droite.

5. Procédé selon la revendication 4, dans lequel l'oléfine concernée est une oléfine à channe droite en C₄.

6. Procédé selon la revendication 1, dans lequel la surface spécifique du support est de 50 m²/g à 1200 m²/g.

7. Procédé selon la revendication 1, dans lequel le support est choisi parmi le charbon végétal, le graphite et les oxydes métalliques.

8. Procédé selon la revendication 7, dans lequel l'oxyde métallique est choisi parmi l'alumine gamma, le dioxyde de titane sous la forme anatase ou rutile, la silice et les mélanges silice-alumine existant à l'état naturel ou synthétiques.

9. Procédé selon la revendication 1, dans lequel le trifluorométhanesulfonate est présent, dans le catalyseur fixé sur support, en une proportion de 1 % à 50 % en poids.

10. Procédé selon la revendication 9, dans lequel le trifluorométhanesulfonate est présent, dans le catalyseur fixé sur support, en une proportion de 5 % à 40 % en poids.

11. Procédé selon la revendication 10, dans lequel le trifluorométhanesulfonate est présent, dans le catalyseur fixé sur support, en une proportion de 10 % à 30 % en poids.

12. Procédé selon la revendication 1, dans lequel le métal du trifluorométhanesulfonate est choisi parmi le magnésium, le baryum, l'aluminium, le gallium, l'étain, le plomb, l'antimoine, le bismuth, le scandium, le chrome, le fer, le cobalt, le nickel, le cuivre, le zinc, l'yttrium, le zirconium, le molybdène, le cadmium et le cérium.

13. Procédé selon la revendication 1, dans lequel le trifluorométhanesulfonate est le trifluorométhanesulfonate d'aluminium.

14. Procédé selon la revendication 1, dans lequel l'halogènure-trifluorométhanesulfonate est un halogénuretrifluorométhanesulfonate d'aluminium.

15. Procédé selon la revendication 1, dans lequel l'oléfine concernée est introduite dans le réacteur d'oligomérisation sous la forme d'un mélange avec un hydrocarbure paraffinique jouant le rôle de diluant.

16. Système catalytique pour catalyse en phase hétérogène, pour l'oligomérisation d'oléfines, **caractérisé** en ce qu'il comprend un trifluorométhanesulfonate ou un halogénure-trifluorométhanesulfonate d'un métal choisi parmi les métaux du groupe II, du groupe III, du groupe IV, du groupe V et du groupe des métaux de transition du Tableau Périodique, ou un mélange quelconque de ces composés, et est fixé, sous forme dispersée, dans toute la masse d'un support solide, chimiquement et thermiquement stable, ayant une surface spécifique de 5 m²/g à 2000 m²/g.

17. Système catalytique selon la revendication 16, dans lequel l'oxyde métallique est choisi parmi l'alumine gamma, le dioxyde de titane sous la forme anatase ou rutile, la silice et les mélanges silice-alumine existant à l'état naturel ou synthétiques.

18. Système catalytique selon la revendication 16, dans lequel le métal est l'aluminium.

## Revendications (Revendications pour l'(les) Etat(s) contractant(s) suivant(s): ES)

1. Procédé d'oligomérisation d'oléfines en C₃ à C₆, comprenant l'oligomérisation de l'oléfine concernée en présence d'un catalyseur acide, en phase hétérogène, à une température de 50°C à 200°C et sous une pression de 0,4 MPa (4 atm) à 10 MPa (100 atm), **caractérisé** en ce que ledit catalyseur est un système comprenant un trifluorométhanesulfonate ou un halogénure-trifluorométhanesulfonate d'un métal choisi parmi les métaux du groupe II, du groupe III, du groupe IV, du groupe V et du groupe des métaux de transition du Tableau Périodique, ou un mélange quelconque de ces composés, et est fixé, sous forme dispersée, dans toute la masse d'un support solide, chimiquement et thermiquement stable, ayant une surface spécifique de 5 m²/g à 2 000 m²/g.

2. Procédé selon la revendication 1, dans lequel la température de la réaction d'oligomérisation est de 100°C à 170°C.

3. Procédé selon la revendication 1, dans lequel on effectue la réaction d'oligomérisation sous une pression de 0,5 MPa à 6 MPa (5 atm à 60 atm).

4. Procédé selon la revendication 1, dans lequel l'oléfine concernée est une oléfine à channe droite.

5. Procédé selon la revendication 4, dans lequel l'oléfine concernée est une oléfine à chaîne droite en C₄.

6. Procédé selon la revendication 1, dans lequel la surface spécifique du support est de 50 m²/g à 1200 m²/g.

7. Procédé selon la revendication 1, dans lequel le support est choisi parmi le charbon végétal, le graphite et les oxydes métalliques.

8. Procédé selon la revendication 7, dans lequel l'oxyde métallique est choisi parmi l'alumine gamma, le dioxyde de titane sous la forme anatase ou rutile, la silice et les mélanges silice-alumine existant à l'état naturel ou synthétiques.

9. Procédé selon la revendication 1, dans lequel le trifluorométhanesulfonate est présent, dans le catalyseur fixé sur support, en une proportion de 1 % à 50 % en poids.

10. Procédé selon la revendication 9, dans lequel le trifluorométhanesulfonate est présent, dans le catalyseur fixé sur support, en une proportion de 5 % à 40 % en poids.

11. Procédé selon la revendication 10, dans lequel le trifluorométhanesulfonate est présent, dans le catalyseur fixé sur support, en une proportion de 10 % à 30 % en poids.

12. Procédé selon la revendication 1, dans lequel le métal du trifluorométhanesulfonate est choisi parmi le magnésium, le baryum, l'aluminium, le gallium, l'étain, le plomb, l'antimoine, le bismuth, le scandium, le chrome, le fer, le cobalt, le nickel, le cuivre, le zinc, l'yttrium, le zirconium, le molybdène, le cadmium et le cérium.

13. Procédé selon la revendication 1, dans lequel le trifluorométhanesulfonate est le trifluorométhanesulfonate d'aluminium.

14. Procédé selon la revendication 1, dans lequel l'halogènure-trifluorométhanesulfonate est un halogénuretrifluorométhanesulfonate d'aluminium.

15. Procédé selon la revendication 1, dans lequel l'oléfine concernée est introduite dans le réacteur d'oligomérisation sous la forme d'un mélange avec un hydrocarbure paraffinique jouant le rôle de diluant.
